# EUROPÄISCHE PATENTSCHRIFT

(11) **EP 1 572 624 B1**
(45) Veröffentlichungstag und Bekanntmachung des Hinweises auf die Patenterteilung: **30.07.2008**
(21) Anmeldenummer: 03795879.0
(22) Anmeldetag: 10.12.2003
(51) Int. Cl.: C07C 231/12

(54) **VERFAHREN ZUR HERSTELLUNG HELLFARBIGER FETTS UREALKANOLAMIDP OLYALKYLENGLYCOLETHER**
METHOD FOR PRODUCING LIGHT COLOURED POLYALKYLENE GLYCOL DIETHYL ETHER OF FATTY ACID ALCANOLAMINE
PROCEDE POUR PRODUIRE DES ETHERS DE POLYALKYLENEGLYCOL D'ALCANOLAMIDES D'ACIDES GRAS DE COULEUR CLAIRE

(30) Priorität: 19.12.2002 DE 10259405
(43) Veröffentlichungstag der Anmeldung: 14.09.2005
(73) Patentinhaber: Cognis IP Management GmbH, 40589 Düsseldorf (DE)
(72) Erfinder: BEHLER, Ansgar, 46240 Bottrop (DE); CLASEN, Frank, 40721 Hilden (DE)
(86) Internationale Anmeldenummer: PCT/EP2003/013977
(87) Internationale Veröffentlichungsnummer: WO 2004/056743

(56) Entgegenhaltungen:
- EP-A- 1 319 647
- WO-A-92/08690

## Beschreibung

### Gebiet der Erfindung

Die Erfindung befindet sich auf dem Gebiet der nichtionischen oberflächenaktiven Verbindungen und betrifft ein Verfahren zur Herstellung spezieller Verbindungen mit verbesserter Farbqualität und vermindertem Anteil an Nebenprodukten.

### Stand der Technik

Die Herstellung von Alkoxylierungsprodukten der Fettsäurealkanolamide ist seit langem bekannt und wird beispielsweise in dem Übersichtsartikel von Großmann **[**Fette, Seifen, Anstrichmittel, 74(1), 58 (1972**)]** ausführlich behandelt. Üblicherweise erfolgt die Umsetzung der Alkanolamide, vorzugsweise der Monoalkanolamide, mit Ethylen- oder Propylenoxid in Gegenwart alkalischer Katalysatoren, wie beispielsweise tertiären Aminen [vgl. EP 0557462 B1, Berol Nobel)]. Von Nachteil ist jedoch, dass die Umsetzungsprodukte in der Regel stark verfärbt sind und mitunter hohe Gehalte an unerwünschten Nebenprodukten, insbesondere an Dioxan enthalten. Beide Faktoren limitieren den Einsatz der Produkte insbesondere für kosmetische Anwendungen.

Die Aufgabe der vorliegenden Erfindung hat daher darin bestanden, ein verbessertes Verfahren zur Alkoxylierung von Fettsäurealkanolamiden zur Verfügung zu stellen, das die oben geschilderten Nachteile zuverlässig vermeidet. Insbesondere sollten die Produkte über eine hohe Farbqualität und einen niedrigen Gehalt an unerwünschten Nebenprodukten, speziell an Dioxan aufweisen.

### Beschreibung der Erfindung

Gegenstand der Erfindung ist ein Verfahren zur Herstellung von hellfarbigen Fettsäurealkanolamidpolyalkylenglycolethern durch Anlagerung von Alkylenoxiden an Fettsäurealkanolamide in Gegenwart alkalischer Katalysatoren, welches sich dadurch auszeichnet, dass man die Alkoxylierung in Gegenwart von Reduktionsmitteln durchführt und die auf diesem Wege erhaltenen Reaktionsprodukte anschließend einer Wasserdampfbehandlung unter alkalischen Bedingungen unterwirft.

Überraschenderweise wurde gefunden, dass die Kombination einer Alkoxylierung in Gegenwart von Reduktionsmitteln mit einer Wasserdampfnachbehandlung unter alkalischen Bedingungen alkoxylierte Fettsäurealkanolamide liefert, die sowohl besonders hellfarbig als auch arm an unerwünschten Nebenprodukten sind. Nicht zu erwarten war insbesondere das Erfordernis einer Dämpfung bei hohen pH-Werten, da üblicherweise Wasserdampfbehandlungen von wässrigen Tensiden im neutralen Bereich durchgeführt werden. Im Gegensatz zu dieser Erfahrung wurde beobachtet, dass die Dämpfung bei pH = 6 bis 7 zu einer signifikanten Farbverschlechterung führt.

### Fettsäurealkanolamide

Die Auswahl der eingesetzten Fettsäurealkanolamide, bei denen es sich um Kondensationsprodukten von technischen Fettsäuren mit Mono- oder Dialkanolaminen handelt, ist an sich unkritisch. Typischer Weise gelangen als Edukte solche Fettsäurealkanolamide zum Einsatz, die der Formel (I) folgen, 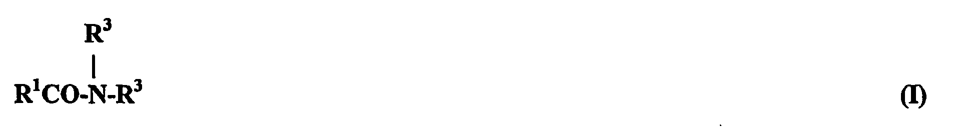 in der R¹CO für einen linearen oder verzweigten, gesättigten oder ungesättigten Acylrest mit 6 bis 22 Kohlenstoffatomen und 0 oder 1 bis 3 Doppelbindungen, R² für eine Hydroxyalkylgruppe mit 2 bis 4 Kohlenstoffatomen und R³ für Wasserstoff oder R² steht. Typische Beispiele sind die Kondensationsprodukte von Capronsäure, Caprylsäure, Caprinsäure, Laurinsäure, Myristinsäure, Palmitinsäure, Stearinsäure, Isostearinsäure, Ölsäure, Linolsäure, Linolensäure, Petroselinsäure, Elaeostearinsäure, 12-Hydroxystearinsäure, Ricinolsäure, Gadoleinsäure, Arachidonsäure, Behensäure, Erucaäsure sowie deren technische Gemische, insbesondere Kokosfettsäure, Palmkernfettsäure, Palmfettsäure und Talgfettsäure mit Monoethanolamin, Diethanolamin, Monopropanolamin und Dipropanolamin sowie deren Gemischen. Vorzugsweise werden Kondensationsprodukte von Kokos- oder Talgfettsäuren mit Monoethanolamin eingesetzt.

### Alkylenoxide

Als Alkylenoxide kommen Ethylenoxid, Propylenoxid, Butylenoxid oder deren Gemische in Frage, wobei die Anlagerung blockweise oder randomisiert erfolgen kann. Üblicherweise werden die Fettsäurealkanolamide und die Alkylenoxide im molaren Verhältnis 1 : 1 bis 1 : 25, vorzugsweise 1 : 2 bis 1 : 10 eingesetzt.

### Alkalische Katalysatoren

Als alkalische Katalysatoren eignen sich neben den Alkalihydroxiden und -carbonaten, vor allem auch Alkoholate, insbesondere Natriummethylat, Natriumethylat oder Kalium-tert.butylat. Wie schon eingangs erwähnt, kommen für diesen Zweck auch tertiäre Amine in Frage. Typischerweise beträgt die Einsatzmenge der alkalischen Katalysatoren 0,1 bis 5, vorzugsweise 0,5 bis 2 Gew.-% - bezogen auf die Einsatzstoffe.

### Reduktionsmittel

Als Reduktionsmittel kommen die Stoffe in Frage, die unter dieser Bezeichnung bekannt sind, beispielsweise Borhydride, insbesondere Natriumborhydrid, sowie hypophosphorige Säure oder deren Alkalisalze in Betracht. Die Einsatzmenge beträgt in der Regel von 0,1 bis 2,5, vorzugsweise 0,2 bis 1 Gew.-% - bezogen auf die Einsatzstoffe.

### Alkoxylierung

Die Alkoxylierung der Fettsäurealkanolamide kann in an sich bekannter Weise durchgeführt werden. In der Regel benutzt man Rührautoklaven, die durch abwechselndes Ausheizen, Evakuieren und Stickstoffaufgabe von anhaftenden Wasserspuren sowie Luftsauerstoff befreit werden. Die Amide werden zusammen mit dem Katalysator und dem Reduktionsmittel vorgelegt und auf eine Temperatur vorzugsweise von 80 bis 150 °C, bevorzugt von 110 bis 140 °C aufgeheizt. Das Alkylenoxid wird anschließend portionsweise bei einem Druck im Bereich von 1 bis 10, vorzugsweise 3 bis 6 bar aufgepresst, wobei es sich empfiehlt, nach Beendigung der Zugabe noch eine ein- bis zweistündige Nachreaktionszeit anzuschließen, wobei das Temperaturniveau allmählich gesenkt werden kann. Die Reaktionsprodukte zeigen nach der Alkoxylierung typischerweise eine Gardner-Farbzahl von 3 bis 4.

### Wasserdampfbehandlung

Nach Abkühlung und Entspannung des Ansatzes werden die rohen Reaktionsprodukte einer Wasserdampfbehandlung unterworfen, bei der es entscheidend ist, dass zuvor ein alkalischer pH-Wert, vorzugsweise pH = 9 bis 12 eingestellt wird. Dies geschieht beispielsweise durch Zugabe einer wässrigen Alkalibase. Anschließend wird bei 100 bis 120 °C und unter ständigem Rühren solange Wasserdampf durch den Ansatz geleitet, bis etwa 10 bis 25 Gew.-% der eingesetzten Dampfmenge als Kondensat anfällt. Dies entspricht typisch einer Behandlung über einen Zeitraum von ca. 60 min. Danach wird das Alkoxylierungsprodukt getrocknet, das typischerweise nunmehr eine Gardner-Farbzahl von unter 2 und einen Dioxangehalt von weniger als 1 ppm aufweist.

### Beispiele

### Beispiel 1

### Herstellung von Kokosfettsäuremonoethanolamid+4EO

In einem 5-1-Rührautoklaven wurden 2929,3 g (entsprechend 11,75 Mol) eines C₈-C₁₈-Kokosfettsäuremonoethanolamids zusammen mit 25 g (entsprechend 0,85 Gew.-% - bezogen auf die Ausgangsverbindung) einer 30 Gew.%igen methanolischen Lösung von Natriummethylat und 5,0 g einer 50 Gew.-%igen wässrigen Lösung von Hypophosphorsäure (entsprechend 0,17 Gew.-% bezogen auf die Ausgangsverbindung) vorgelegt. Der Behälter wurde 30 min bei 80 °C evakuiert und anschließend mit Stickstoff belüftet. Anschließend wurde die Mischung auf 110 °C erhitzt und bei einem Druck von bis zu 5 bar portionsweise 2068,0 g (entsprechend 47 Mol) Ethylenoxid aufgepresst. Die Reaktionszeit betrug 90 min; anschließend wurde 60 min bei 110 °C und 30 min bei 80 °C nachgerührt. Nach dem Abkühlen und entspannen wurde das ethoxylierte Fettsäuremonoethanolamid als klare Flüssigkeit (Farbzahl nach Gardner 3,5; Hydroxylzahl 168) erhalten.

### Vergleichsbeispiel V1

### Herstellung von Kokosfettsäuremonoethanolamid+4EO

Beispiel 1 wurde wiederholt, jedoch auf die Mitverwendung von Hypophosphorsäure verzichtet. Das resultierende ethoxylierte Fettsäuremonoethanolamid zeigte eine Farbzahl nach Gardner von 3,9 und eine Hydroxylzahl von 164.

### Beispiel 2

### Dämpfung von Kokosfettsäuremonoethanolamid+4EO

1000 g des nach Beispiel 1 hergestellten Kokosfettsäuremonoethanolamid+4EO wurden mit Hilfe von wässriger Natriumhydroxidlösung auf einen pH-Wert von ca. 11 eingestellt und in einer 5-1-Rührapparatur vorgelegt. Bei 120 °C wurde solange unter Rühren Wasserdampf durch das Ethoxylat geleitet, bis - bezogen auf den Einsatzstoff - 20 Gew.-% Wasser kondensiert waren (dauerte 60 Minuten). Anschließend wurde das Produkt bei 120 °C und 30 mbar getrocknet. Das Endprodukt besaß eine Gardner-Farbzahl von 1,1 und einen Dioxangehalt unter 1 ppm.

### Vergleichsbeispiel V2

### Dämpfung von Kokosfettsäuremonoethanolamid+4EO

1000 g des nach Beispiel 1 hergestellten Kokosfettsäuremonoethanolamid+4EO wurden auf einen neutralen pH-Wert von 6,5 eingestellt und in einer 5-1-Rührapparatur vorgelegt. Bei 120 °C wurde solange unter Rühren Wasserdampf durch das Ethoxylat geleitet, bis - bezogen auf den Einsatzstoff - 20 Gew.% Wasser kondensiert waren (dauerte 60 Minuten). Anschließend wurde das Produkt bei 120 °C und 30 mbar getrocknet. Das Endprodukt besaß eine Gardner-Farbzahl von 6,5 und einen Dioxangehalt unter 1 ppm.

## Patentansprüche

1. Verfahren zur Herstellung von hellfarbigen Fettsäurealkanolamidpolyalkylenglycolethem durch Anlagerung von Alkylenoxiden an Fettsäurealkanolamide in Gegenwart alkalischer Katalysatoren, **dadurch gekennzeichnet, dass** man die Alkoxylierung in Gegenwart von Reduktionsmitteln durchführt und die auf diesem Wege erhaltenen Reaktionsprodukte anschließend einer Wasserdampfbehandlung unter alkalischen Bedingungen unterwirft.

2. Verfahren nach Anspruch 1, **dadurch gekennzeichnet, dass** man Fettsäurealkanolamide der Formel (**I**) einsetzt, 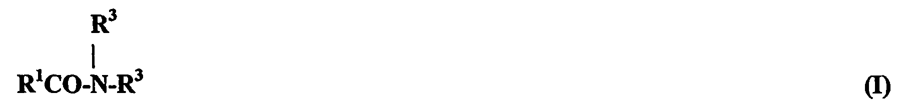 in der R¹CO für einen linearen oder verzweigten, gesättigten oder ungesättigten Acylrest mit 6 bis 22 Kohlenstoffatomen und 0 oder 1 bis 3 Doppelbindungen, R² für eine Hydroxyalkylgruppe mit 2 bis 4 Kohlenstoffatomen und R³ für Wasserstoff oder R² steht.

3. Verfahren nach den Ansprüchen 1 und/oder 2, **dadurch gekennzeichnet, dass** man als Alkylenoxide Ethylenoxid, Propylenoxid, Butylenoxid oder deren Gemische einsetzt.

4. Verfahren nach mindestens einem der Ansprüche 1 bis 3, **dadurch gekennzeichnet, dass** man die Fettsäurealkanolamide und die Alkylenoxide im molaren Verhältnis 1 : 1 bis 1 : 25 einsetzt.

5. Verfahren nach mindestens einem der Ansprüche 1 bis 4, **dadurch gekennzeichnet, dass** man die alkalischen Katalysatoren in Mengen von 0,1 bis 5 Gew.% - bezogen auf die Einsatzstoffe - einsetzt.

6. Verfahren nach mindestens einem der Ansprüche 1 bis 5, **dadurch gekennzeichnet, dass** man als Reduktionsmittel Natriumborhydrid, hypophosphorige Säure oder deren Alkalisalze einsetzt.

7. Verfahren nach mindestens einem der Ansprüche 1 bis 6, **dadurch gekennzeichnet, dass** man die Reduktionsmittel in Mengen von 0,1 bis 2,5 Gew.-% - bezogen auf die Einsatzstoffe - einsetzt.

8. Verfahren nach mindestens einem der Ansprüche 1 bis 7, **dadurch gekennzeichnet, dass** man die Alkoxylierung bei Temperaturen im Bereich von 80 bis 150 °C durchführt.

9. Verfahren nach mindestens einem der Ansprüche 1 bis 8, **dadurch gekennzeichnet, dass** man die Alkoxylierung bei Drücken im Bereich von 1 bis 10 bar durchführt.

10. Verfahren nach mindestens einem der Ansprüche 1 bis 9, **dadurch gekennzeichnet, dass** man die Wasserdampfbehandlung bei einem pH-Wert von 9 bis 12 durchführt.

## Claims

1. A process for the production of light-colored fatty acid alkanolamide polyalkylene glycol ethers by addition of alkylene oxides onto fatty acid alkanolamides in the presence of alkaline catalysts, **characterized in that** the alkoxylation is carried out in the presence of reducing agents and the reaction products obtained in this way are subjected to a treatment with steam under alkaline conditions.

2. A process as claimed in claim 1, **characterized in that** the fatty acid alkanolamides used correspond to formula (I): 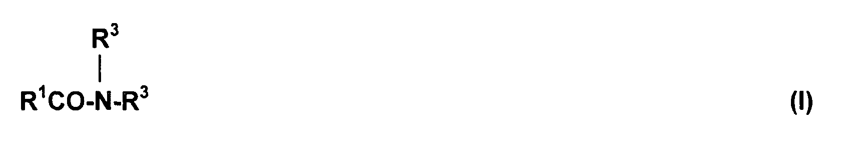 where R¹CO is a linear or branched, saturated or unsaturated acyl group containing 6 to 22 carbon atoms and 0 or 1 to 3 double bonds, R² is a hydroxyalkyl group containing 2 to 4 carbon atoms and R³ is hydrogen or has the same meaning as R².

3. A process as claimed in claims 1 and/or 2, **characterized in that** ethylene oxide, propylene oxide, butylene oxide or mixtures thereof are used as the alkylene oxide.

4. A process as claimed in at least one of claims 1 to 3, **characterized in that** the fatty acid alkanolamides and the alkylene oxides are used in a molar ratio of 1:1 to 1:25.

5. A process as claimed in at least one of claims 1 to 4, **characterized in that** the alkaline catalysts are used in quantities of 0.1 to 5% by weight, based on the starting materials.

6. A process as claimed in at least one of claims 1 to 5, **characterized in that** sodium borohydride, hypophosphorous acid or alkali metal salts thereof are used as the reducing agent.

7. A process as claimed in at least one of claims 1 to 6, **characterized in that** the reducing agents are used in quantities of 0.1 to 2.5% by weight, based on the starting materials.

8. A process as claimed in at least one of claims 1 to 7, **characterized in that** the alkoxylation is carried out at temperatures of 80 to 150°C.

9. A process as claimed in at least one of claims 1 to 8, **characterized in that** the alkoxylation is carried out under pressures of 1 to 10 bar.

10. A process as claimed in at least one of claims 1 to 9, **characterized in that** the treatment with steam is carried out at a pH value of 9 to 12.

## Revendications

1. Procédé d'obtention d'éthers de polyalkylèneglycol d'alcanolamides, d'acides gras, de couleur claire par addition d'oxydes d'alkylène sur des alcanolamides d'acides gras en présence de catalyseurs alcalins,
**caractérisé en ce qu'**
l'on effectue l'alkoxylation en présence d'agents de réduction puis on soumet les produits de réaction ainsi obtenus à un traitement par la vapeur d'eau dans des conditions alcalines.

2. Procédé selon la revendication 1,
**caractérisé en ce qu'**
l'on met en oeuvre des alcanolamides d'acides gras de formule (I) 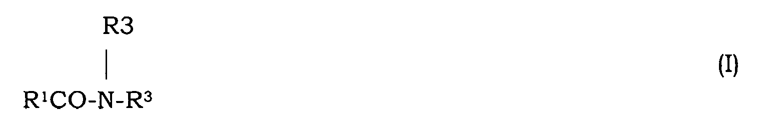 dans laquelle R¹CO représente un radical acyl linéaire ou ramifié, saturé ou insaturé ayant de 6 à 22 atomes de carbone et 0 ou de 1 à 3 doubles liaisons, R² représente un groupe hydroxyalkyl ayant de 2 à 4 atomes de carbone et R³ représente l'hydrogène ou R².

3. Procédé selon les revendications 1 et/ou 2,
**caractérisé en ce qu'**
en tant qu'oxydes d'alkylène, on met en oeuvre de l'oxyde d'éthylène, de l'oxyde de propylène, de l'oxyde de butylène ou leurs mélanges.

4. Procédé selon au moins l'une des revendications 1 à 3,
**caractérisé en ce qu'**
on met en oeuvre les alcanolamides d'acides gras et les oxydes d'alkylène dans un rapport molaire de 1:1 à 1:25.

5. Procédé selon au moins l'une des revendications 1 à 4,
**caractérisé en ce qu'**
l'on met en oeuvre les catalyseurs alcalins en quantité de 0,1 à 5 % en poids par rapport aux matières utilisées.

6. Procédé selon au moins l'une des revendications 1 à 5,
**caractérisé en ce qu'**
en tant qu'agent de réduction on met en oeuvre du borohydrure de sodium, de l'acide hypophosphoreux ou ses sels alcalins.

7. Procédé selon au moins l'une des revendications 1 à 6,
**caractérisé en ce qu'**
on met en oeuvre l'agent de réduction en quantité de 0,1 à 2,5 % en poids par rapport aux matières utilisées.

8. Procédé selon au moins l'une des revendications 1 à 7,
**caractérisé en ce qu'**
on met en oeuvre l'alkoxylation à des températures situées dans la plage de 80 à 150°C.

9. Procédé selon au moins l'une des revendications 1 à 8,
**caractérisé en ce qu'**
on met en oeuvre l'alkoxylation sous des pressions situées dans la plage de 1 à 10 bars.

10. Procédé selon au moins l'une des revendications 1 à 9,
**caractérisé en ce qu'**
on met en oeuvre le traitement par la vapeur d'eau dans une plage de pH de 9 à 12.
